# EUROPÄISCHE PATENTSCHRIFT

(11) **EP 0 584 321 B1**
(45) Veröffentlichungstag und Bekanntmachung des Hinweises auf die Patenterteilung: **26.09.2001**
(21) Anmeldenummer: 93905133.0
(22) Anmeldetag: 15.03.1993
(51) Int. Cl.: G01N 21/89, G01N 33/36

(54) **VERFAHREN UND VORRICHTUNG ZUR DETEKTION VON VERUNREINIGUNGEN IN EINEM TEXTILEN PRÜFGUT**
PROCESS AND DEVICE FOR DETECTING IMPURITIES IN A TEXTILE TEST SAMPLE
PROCEDE ET DISPOSITIF DE DETECTION D'IMPURETES DANS UN ECHANTILLON TEXTILE DE CONTROLE

(30) Priorität: 17.03.1992 CH 85592
(43) Veröffentlichungstag der Anmeldung: 02.03.1994
(73) Patentinhaber: ZELLWEGER LUWA AG, 8610 Uster (CH)
(72) Erfinder: JOSS, Rolf, CH-8810 Horgen (CH); WAMPFLER, Hans, CH-8049 Zürich (CH)
(86) Internationale Anmeldenummer: CH9300071
(87) Internationale Veröffentlichungsnummer: WO9319359

(56) Entgegenhaltungen:
- EP-A- 0 271 728
- CH-A- 424 316
- CH-A- 674 379
- GB-A- 2 064 106
- PATENT ABSTRACTS OF JAPAN vol. 10, no. 110 (P-450)(2167) 24. April 1986 & JP,A,60 239 653 (FURUKAWA DENKI KOGYO) 28 November 1985

## Beschreibung

Die vorliegende Erfindung betrifft ein Verfahren zur Detektion von Verunreinigungen in einem textilen Prüfgut von der Art eines Garnes, Vorgarnes oder Bandes, bei welchem das Prüfgut mit Licht beaufschlagt, das vom Prüfgut reflektierte Licht gemessen und aus einer Aenderung des reflektierten Lichts auf das Vorhandensein einer Verunreinigung geschlossen wird.

Bei einem aus der EP-B-0 197 763 bekannten Verfahren dieser Art ist ein das Prüfgut in der Art eines Führungsschlitzes umgebender Hintergrund vorgesehen, welcher ebenfalls mit Licht beaufschlagt ist. Der Hintergrund ist dabei so auf das Prüfgut abgestimmt, dass die Gesamtmenge des vom Prüfgut reflektierten und des vom Hintergrund herrührenden Lichts von den Abmessungen und der Dichte des Prüfguts und von der Verteilung der Fasern innerhalb von diesem unabhängig ist. Auf diese Weise soll es möglich sein, dass eine Veränderung im reflektierten Licht eine Verunreinigung anzeigt und nicht eine Veränderung in den Dimensionen, in der Dichte oder in der Faserverteilung im Textilerzeugnis.

Abgesehen davon, dass bei diesem Verfahren bei jedem Wechsel der Art oder des Typs des Prüfguts relativ aufwendige Einstellungsarbeiten zur Anpassung des Hintergrunds an das Prüfgut erforderlich sind, ist dieses Verfahren auch sehr empfindlich auf Verschmutzung und Alterung des Hintergrunds. Und beides sind Phänomene, die sich in einem Textilbetieb nicht vermeiden lassen und gerade dort ständig auftren.

Aus der EP-A-0 271 728 ist ein Verfahren und eine Anordnung zur Messung und Ueberwachung von Eigenschaften von Garnen oder Seilen bekannt, bei denen vom Garn oder Seil ein zweidimensionales Bild aufgenommen und in elektrische Bildsignale umgewandelt wird. Dabei können auch mehrere Bildsensoren räumlich so angeordnet sein, dass Angaben über die dreidimensionale Geometrie, insbesondere die Rundheit gewonnen werden können.

Allerdings ist es damit nicht möglich Verunreinigungen, insbesondere Fremdstoffe in einem Garn von blossen Durchmesseränderungen zu unterscheiden, die solche Fremdstoffe fast zwangsläufig bewirken.

Durch die Erfindung soll ein Verfahren und eine Vorrichtung angegeben werden, die eindeutige Detektion von Verunreinigungen in einem textilen Prüfgut ermöglichen und welche keine Abstimmung des Hintergrundes auf das Prüfgut erfordern und bei denen daher irgendwelche durch den Hintergrund verursachte Störungen ausgeschlossen sind.

Diese Aufgabe wird erfindungsgemäss dadurch gelöst, dass das Prüfgut von mindestens zwei Lichtquellen beleuchtet und zusätzlich zum Oberflächensignal ein für den Durchmesser des Prüfguts oder dessen Aenderung charakteristisches Durchmessersignal gewonnen wird, und dass die so erhaltenen Messsignale miteinander verknüpft werden.

Die Erfindung geht also davon aus, dass das von einem gegebenen Prüfgut eines gegebenen Durchmessers reflektierte Licht so lange konstant ist, als sich der Reflexionsgrad des Prüfguts nicht ändert, dieses also keine Verunreinigungen enhält. Die Aenderung des Reflexionsgrades und damit das Vorhandensein von Verunreinigungen, insbesondere von Fremdfasern, kann nun dadurch detektiert werden, dass man aus dem Oberflächen- und aus dem Durchmessersignal eine Grösse bildet und diese untersucht.

Entsprechend ist eine bevorzugte Ausführungsform des erfindungsgemässen Verfahrens dadurch gekennzeichnet, dass aus dem Reflexions- und aus dem Durchmessersignal ein Quotient abgeleitet und fortlaufend auf Abweichungen von einem Mittelwert überprüft wird.

Die Erfindung betrifft weiter eine Vorrichung zur Durchführung des genannten Verfahrens, mit Sendemitteln zur Beleuchtung des Prüfguts, mit Empfangsmitteln zur Messung des vom Prüfgut reflektierten und/oder absorbierten Lichts und mit Mitteln zur Auswertung der Messsignale.

Die erfindungsgemässe Vorrichtung ist dadurch gekennzeichnet, dass die Sendemittel so ausgebildet sind, dass eine simultane Beleuchtung mehrerer auf einem gleichen Abschnitt des Prüfguts liegender Stellen oder eine mehrmalige Beleuchtung des Prüfguts an der gleichen Stelle erfolgt, und dass Empfangsmittel für das vom Prüfgut an den genannten Stellen reflektierte und/oder abgeschwächte Licht vorgesehen sind.

Nachfolgend wird die Erfindung anhand von drei Ausführungsbeispielen und der Zeichnungen näher erläutert; es zeigt:
- Fig. 1: eine schematische Darstellung eines ersten Ausführungsbeispiels einer erfindungsgemässen Vorrichtung,
- Fig. 2: eine schematische Darstellung eines zweiten Ausführungsbeispiels; und
- Fig. 3: eine Darstellung eines dritten Ausführungsbeispiels einer erfindungsgemässen Vorrichtung.

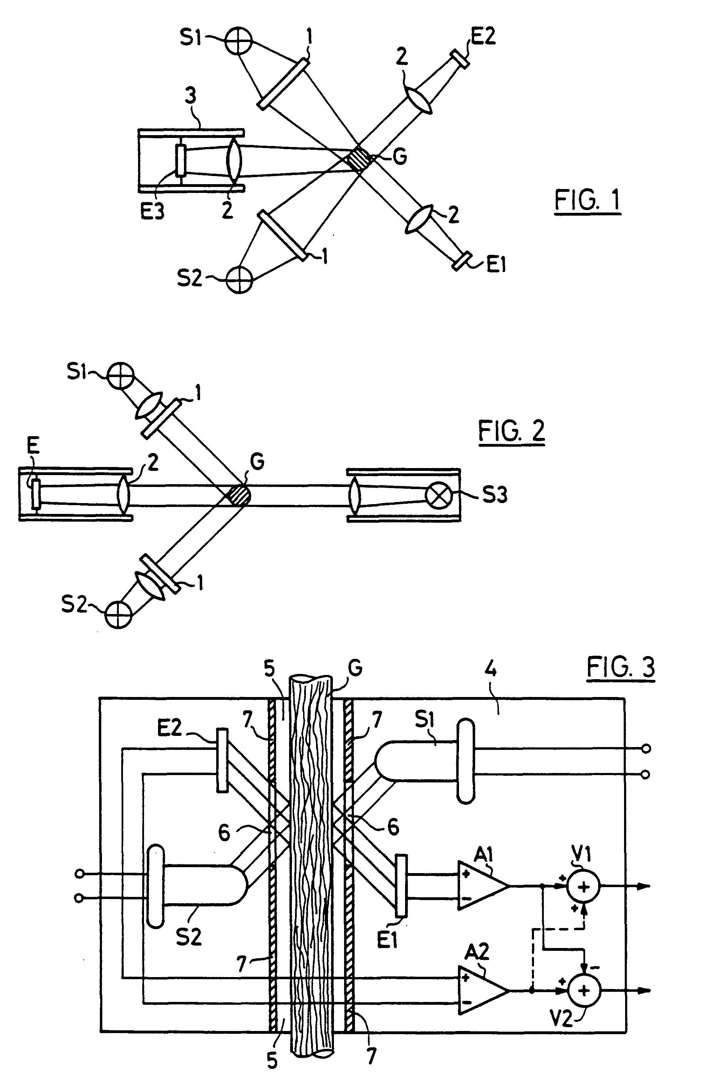

Bei den in den Fig. 1 und 2 dargestellten Ausführungsbeispielen erfolgt eine simultane Messung des Garndurchmessers und der Reflexion an einem Garn G, wobei sich die Messsignale auf die gleiche Garnstelle beziehen. Das Messfeld ist nicht grösser als 0,5 mm. Dies deswegen, weil eine typische Fremdfaser um das Garn herumgewunden ist, und ein typischer Wert für die Garndrehung bei 1000 Drehungen pro Meter liegt. Anstatt der simultanen Messung an einer Garnstelle kann man auch an zwei Stellen X und Y messen, das Ergebnis der früheren Stelle X speichern, und die Messungen so synchronisieren, dass an der Stelle Y genau dann gemessen wird, wann diese von dem vorher bei X gemessenen Messfeld des Garns durchlaufen wird.

Aus diesen beiden Signalen für die Reflexion und den Durchmesser wird ein Quotient gebildet, der, im Unterschied zu der zum Garndurchmesser proportionalen Reflexion, bei gleichbleibendem Reflexionsgrad des Garns unabhängig von eventuellen Durchmesserschwankungen ist. Da der Reflexionsgrad vom Fasermaterial abhängig ist, bleibt der genannte Quotient so lange konstant, bis eine Störung mit einem anderen Reflexionsgrad als das Fasermaterial, also beispielsweise eine Fremdfaser, die Messstelle passiert. Der Wert des Quotienten Reflexion durch Durchmesser kann durch laufende Mittelwertbildung über längere Garnstücke gefunden werden. Diesem Referenzwert werden entsprechende Trigger-Schwellenwerte zugeordnet, deren Ueberschreiten durch das Messsignal jeweils eine Fremdfaser oder eine andere Verunreinigung anzeigt, welche heller oder dunkler als das betrachtete Fasermaterial ist.

Die Messung des Durchmessers erfolgt vorzugsweise ebenso wie die Reflexionsmessung optisch, und zwar im Auflicht, oder im Durchlicht durch eine Abschattungsmethode. Im letzteren Fall ist eine leicht diffuse Beleuchtung besser geeignet als eine parallele Lichtführung, da dadurch der Intensitätsabfall am Rand des Garns besser kompensiert wird. Vorzugsweise wird eine grossflächige diffuse Beleuchtung verwendet, durch welche die gekrümmte Garnoberfläche aus einem möglichst grossen Raumwinkel heraus gleichmässig beleuchtet wird.

Selbstverständlich kann der Durchmesser des Garns auch kapazitiv gemessen werden, wobei dann vorzugsweise ein kombinierter Messkopf der in der EP-A-0 401 600 beschriebenen Art verwendet wird. Man kann die Messanordnung auch so modifizieren, dass zusätzlich noch weitere Parameter, wie zum Beispiel die Haarigkeit, gemessen werden. In diesem Fall empfiehlt sich zur Kompensation des Intensitätsabfalls am Rand des Garns eine zusätzliche Dunkelfeldbeleuchtung, wie sie beispielsweise in der EP-A-0 226 843 beschrieben ist.

Weil die Kontraste in kurzwelligem Licht im allgemeinen deutlicher sichtbar sind als in langwelligem, wird vorzugsweise kurzwelliges Licht verwendet. Andererseits gibt es auch Fasern, bei denen die am häufigsten auftretenden Fremdfasern eine hohe Absorbtion im Infrarotbereich haben. In diesen Fällen ist es sinnvoll, entsprechend langwelliges Licht zu verwenden. Als Lichtquellen werden vorzugsweise Leuchtdioden verwendet, weil diese langlebig und stabil sind und sich ausserdem gut modulieren lassen. Als Empfänger werden Fotodioden oder Fotomultiplier verwendet.

Bei dem in Fig. 1 dargestellten Ausführungsbeispiel sind zwei Sender S1 und S2 und drei Empfänger E1, E2 und E3 vorgesehen, wobei die Empfänger E1 und E2 den Durchmesser des Garnes G messen und der Empfänger E3 die Reflexion. Mit dem Bezugszeichen 1 sind im Strahlengang kurz nach den Sendern angeordnete Streuscheiben und mit dem Bezugszeichen 2 sind im Strahlengang kurz vor den Empfängern angeordnete Sammellinsen bezeichnet. Mindestens der Empfänger E3 für das vom Garn G reflektierte Licht ist zur Abschirmung von störenden Umgebungseinflüssen wie Fremdlicht und dergleichen in einem Gehäuse 3 eingebaut.

Beim Ausführungsbeispiel von Fig. 2 werden drei Sender S1, S2 und S3, aber nur ein Empfänger E verwendet. Die Sender S1 und S2 beleuchten das Garn G mit diffusem Licht und der Empfänger E misst einerseits das Reflexionslicht und andererseits das von der Lichtquelle S3 kommende Durchlicht, welches zur Durchmesserbestimmung verwendet wird. Die Trennung der beiden gleichzeitig anfallenden Lichtarten erfolgt entweder durch ein Zeitmultiplexverfahren oder durch Modulation, wobei im letzteren Fall die Sender S1 und S2 für das Reflexionslicht anders moduliert werden als der Sender S3 für das Durchlicht und die Anteile durch entsprechende Demodulation getrennt werden.

Bei dem in Fig. 3 dargestellten Ausführungsbeispiel sind zwei Reflexionslichtschranken S1, E1 und S2, E2 vorgesehen, welche das Garn G an einander gegenüberliegenden Seiten abtasten. Die Lichtschranken sind zu beiden Seiten eines in einem Gehäuse 4 gebildeten und vom Garn G durchlaufenen Messschlitzes oder Messspalts 5 vorgesehen, welcher im Bereich der Lichtschranken optisch durchsichtige Fenster 6 für den Durchtritt der Lichtstrahlen aufweist. Die beiden Lichtschranken S1, E1 und S2, E2 sind so angeordnet, dass jeweils der Sender der einen dem Empfänger der anderen Lichtschranke gegenüber liegt. Eventuelles Durchlicht zwischen den beiden Lichtschranken, also zwischen S1 und E2 und/oder zwischen S2 und E1 wird durch die Dimensionierung der Fenster 6 verhindert. Oder mit anderen Worten, die oben und unten an die Fenster 6 anschliessenden Partien 7 der Seitenwände des Messspalts 5 wirken als Blende zur Verhinderung von Durchlicht vom Sender S1 zum Empfänger E2 beziehungsweise vom Sender S2 zum Empfänger E1.

Jede der beiden Lichtschranken bildet einen Messkanal, dessen Ausgangssignal eine dem Durchmesser des betrachteten Garnes G proportionale Grösse darstellt. Wenn man die Ausgangssignale der beiden Kanäle miteinander verknüpft und das eine Signal vom anderen subtrahiert, dann liefert dieses Subtraktionssignal, wenn es einen um Null liegenden Bandbereich über- oder unterschreitet, einen Hinweis auf das Vorhandensein einer Verunreinigung.

Jeder der beiden Kanäle enthält einen an den jeweiligen Empfänger E1, E2 angeschlossenen Verstärker A1 beziehungsweise A2 und mindestens einer der Kanäle enthält ein an den Verstärker angeschlossenes Verknüpfungsglied zur Verknüpfung der Signale der beiden Kanäle. Dieses Verknüpfungsglied, welches an seinem Ausgang ein Verunreigungssignal liefert, ist in Fig. 3 mit dem Bezugszeichen V2 bezeichnet. Das in dem den Empfänger E1 und den Verstärker A1 enthaltenden Kanal eingezeichnete Verknüpfungsglied V1 ist fakultativ und braucht nicht unbedingt vorhanden zu sein. Wenn es vorhanden ist, dann dient es zur Addition der Ausgangssignale der beiden Kanäle und liefert somit, so wie jeder Kanal für sich alleine, ein dem Garndurchmesser proportionales Signal, aus dem nach entsprechender Eichung der Garndurchmesser abgeleitet werden kann.

Durch Weglassen der Blenden 7 und entsprechende Vergrösserung der Fenster 6 kann zwischen S1 und E2 und/oder zwischen S2 und E1 eine Bestimmung des Garndurchmessers im Durchlicht erfolgen. In diesem Fall würden die beiden Reflexionslichtschranken im Zeitmultiplex arbeiten.

Die anhand der Figuren 1 bis 3 beschriebenen Vorrichtungen zur Detektion von Verunreinigungen in einem Garn ist als kompakter Messkopf ausgebildet und wird vorzugsweise in Kombination mit einem elektronischen Garnreiniger verwendet (siehe dazu die EP-B-0 197 763), dessen Schneidvorrichtung zusätzlich zum Messkopf des Reinigers auch vom Messkopf für die Verunreinigungen angesteuert ist.

Wie schon erwähnt wurde, können mit einer einzigen optischen Abtastvorrichtung mehrere Funktionen ausgeführt werden, also beispielsweise Garnreinigung, Haarigkeitsmessung und Fremdfasererkennung. Ebenso ist es möglich, mit einem kombinierten kapazitiv/optischen Messorgan kapazitiv die Garnfehler für die Garnreinigung und optisch die Haarigkeit und die für Fremdfasern repräsentative Reflexion zu messen.

## Patentansprüche

1. Verfahren zur Detektion von Verunreinigungen in einem textilen Prüfgut (G) von der Art eines Games, Vorgames oder Bandes, bei welchem das Prüfgut mit Licht beaufschlagt, das vom Prüfgut beeinflusste Licht gemessen und aus einer Änderung des gemessenen Lichts auf das Vorhandensein einer Verunreinigung geschlossen wird, **dadurch gekennzeichnet, dass**
an einer gleichen Garnstelle (X, Y) einerseits die Reflexion des Lichts und andererseits der Gamdurchmesser gemessen wird, wobei ein Signal für die Reflexion des Lichts und ein Signal für den Garndurchmesser entsteht, dass
aus den beiden Signalen ein Quotient gebildet wird, dass
aus laufend erzeugten Werten für den Quotienten ein Mittelwert gebildet wird und dass
Abweichungen von dem Mittelwert ermittelt und so eine Verunreinigung oder eine Fremdfaser anzeigen.

2. Verfahren zur Detektion von Verunreinigungen in einem textilen Prüfgut von der Art eines Games, Vorgames oder Bandes, bei welchem das Prüfgut mit Licht beaufschlagt, das vom Prüfgut beeinflusste Licht gemessen und aus einer Änderung des gemessenen Lichts auf das Vorhandensein einer Verunreinigung geschlossen wird, **dadurch gekennzeichnet, dass**
auf gegenüberliegenden Seiten an einer gleichen Garnstelle, die Reflexion des Lichts gemessen und dabei zwei Signale abgeleitet werden, dass
die beiden Signale voneinander subtrahiert werden, so dass ein Subtraktionssignal entsteht und dass
Abweichungen des Subtraktionssignales von einem um Null liegenden Bandbereich eine Verunreinigung oder eine Fremdfaser anzeigen.

3. Verfahren nach Anspruch 1, **dadurch gekennzeichnet, dass** das Durchmessersignal für die Detektion von Garnfehlern für die Garnreinigung verwendet wird.

4. Verfahren nach Anspruch 1, **dadurch gekennzeichnet, dass** das Reflexionssignal für die Messung der Haarigkeit des Prüfguts (G) verwendet wird.

5. Verfahren nach Anspruch 1, **dadurch gekennzeichnet, dass** das Durchmessersignal aus dem von Prüfgut (G) reflektierten Licht gewonnen wird.

6. Verfahren nach Anspruch 1, **dadurch gekennzeichnet, dass** das Durchmessersignal im Durchlicht durch eine Abschattungsmethode gewonnen wird.

7. Verfahren nach Anspruch 2, **dadurch gekennzeichnet, dass** der genannte Mittelwert durch laufende Mittelwertbildung über grössere Längen des Prüfguts (G) adaptiv bestimmt wird.

8. Vorrichtung zur Durchführung des Verfahrens nach Anspruch 2, mit Sendemitteln (S1, S2) zur Beleuchtung des Prüguts (G), mit Empfangsmitteln (E1, E2) zur Messung von reflektiertem Licht und Mitteln zur Auswertung der Messsignale, **dadurch gekennzeichnet, dass**
die Empfangsmittel zur Messung von am Garn reflektiertem Licht, an gegenüberliegenden Seiten des Garns angeordnet sind und dass
die Mittel zur Auswertung der Messsignale ein Verknüpfungsglied (V2) aufweisen, das die Subtraktion von Signalen aus den Empfangsmitteln ermöglicht und an seinem Ausgang ein Verunreinigungssignal liefert.

## Claims

1. Process for detecting impurities in a textile test material (G) of the yarn, roving or sliver type, in which the test material is subjected to light, the light influenced by the test material is measured and the presence of an impurity is concluded from a change of the measured light, **characterised in that** the reflection of the light, on the one hand, and the yarn diameter, on the other hand, are measured at the same yarn point (X, Y), a signal being obtained for the reflection of the light and a signal being obtained for the yarn diameter, in that a quotient is formed from the two signals, in that an average value is formed from continuously generated values for the quotient, and in that deviations from the average value are determined and thus indicate an impurity or a foreign fibre.

2. Process for detecting impurities in a textile test material of the yarn, roving or sliver type, in which the test material is subjected to light, the light influenced by the test material is measured and the presence of an impurity is concluded from a change of the measured light, **characterised in that** the reflection of the light is measured on opposite sides at the same yarn point and at the same time two signals are derived, in that the two signals are subtracted from one another, so that a subtraction signal is obtained, and in that deviations of the subtraction signal from a band range lying around zero indicate an impurity or a foreign fibre.

3. Process according to Claim 1, **characterised in that** the diameter signal is used for detecting yarn faults for the purpose of yarn cleaning.

4. Process according to Claim 1, **characterised in that** the reflection signal is used for measuring the hairiness of the test material (G).

5. Process according to Claim 1, **characterised in that** the diameter signal is obtained from the light reflected by the test material (G).

6. Process according to Claim 1, **characterised in that** the diameter signal is obtained by transmitted light using a shading method.

7. Process according to Claim 2, **characterised in that** said average value is determined adaptively by continuous averaging over relatively large lengths of the test material (G).

8. Device for carrying out the process according to Claim 2, with transmission means (S1, S2) for illuminating the test material (G), with reception means (E1, E2) for measuring reflected light, and with means for evaluating the measurement signals, **characterised in that** the reception means for measuring light reflected on the yarn are arranged on opposite sides of the yarn, and in that the means for evaluating the measurement signals have a logic element (V2) which allows the subtraction of signals from the reception means and delivers an impurity signal at its output.

## Revendications

1. Procédé de détection d'impuretés dans un échantillon textile de contrôle (G) du type de filé, mèche de préparation ou ruban, où l'échantillon de contrôle est exposé à la lumière, la lumière influencée par l'échantillon de contrôle est mesurée et, à partir d'une modification de la lumière mesurée, on conclut sur la présence d'une impureté, **caractérisé**
**en ce que** sont mesurés à un même emplacement de filé (X, Y) d'une part la réflexion de la lumière et d'autre part le diamètre du filé, où sont produits un signal pour la réflexion de la lumière et un signal pour le diamètre du filé,
en ce qu'il est formé à partir des deux signaux un quotient,
en ce qu'il est formé à partir des valeurs produites continuellement pour le quotient une valeur moyenne et
en ce que des écarts par rapport à la valeur moyenne sont déterminés et indiquent ainsi une impureté ou une fibre étrangère.

2. Procédé de détection d'impuretés dans un échantillon textile de contrôle du type de filé, mèche de préparation ou ruban, où l'échantillon de contrôle est exposé à la lumière, la lumière influencée par l'échantillon de contrôle est mesurée et, à partir d'une modification de la lumière mesurée, on conclut sur la présence d'une impureté, **caractérisé**
**en ce que** sont mesurés sur des côtés opposés à un même emplacement de filé, la réflexion de la lumière et, ce faisant, deux signaux sont dérivés,
en ce que les deux signaux sont soustraits l'un de l'autre de maniéré à obtenir un signal de soustraction et
en ce que des écarts du signal de soustraction par rapport à une zone de bande située autour de zéro indiquent une impureté ou une fibre étrangère.

3. Procédé selon la revendication 1, **caractérisé en ce que** le signal de diamètre est utilisé pour la détection de défauts de filé pour l'épuration du filé.

4. Procédé selon la revendication 1, **caractérisé en ce que** le signal de réflexion est utilisé pour la mesure de la pilosité de l'échantillon de contrôle (G).

5. Procédé selon la revendication 1, **caractérisé en ce que** le signal de diamètre est obtenu à partir de la lumière réfléchie par l'échantillon de contrôle (G).

6. Procédé selon la revendication 1, **caractérisé en ce que** le signal du diamètre est obtenu en lumière par transmission selon une méthode d'obscurcissement.

7. Procédé selon la revendication 2, **caractérisé en ce que** la valeur moyenne indiquée est déterminée d'une manière adaptative par une formation continuelle de la valeur moyenne sur de plus grandes longueurs de l'échantillon de contrôle (G).

8. Dispositif pour la mise en oeuvre du procédé selon la revendication 2, avec des moyens d'émission (S1, S2) pour l'éclairage de l'échantillon de contrôle (G), avec des moyens de réception (E1, E2) pour la mesure de la lumière réfléchie et avec des moyens pour l'évaluation des signaux de mesure, **caractérisé**
en ce que les moyens de réception pour la mesure de la lumière réfléchie au filé sont disposés à des côtés opposés du filé et
en ce que les moyens pour l'évaluation des signaux de mesure présentent un élément logique (V2) qui permet la soustraction de signaux à partir des moyens de réception et qui fournit à sa sortie un signal d'impureté.
